# DEMANDE DE BREVET EUROPEEN

(11) **EP 1 813 267 A1**
(43) Date de publication de la demande: **01.08.2007**
(21) Numéro de dépôt: 07300747.8
(22) Date de dépôt: 25.01.2007
(51) Int. Cl.: A61K 8/92, A61Q 1/02, A61Q 1/08

(54) **Composition cosmétique matifiante**

(30) Priorité: 26.01.2006 FR 0650282
(71) Demandeur: L'ORÉAL, 75008 Paris (FR)
(72) Inventeur: Arnaud, Pascal, 94240 l'Hay les roses (FR); Gavache, Nathalie, 45000 Orleans (FR)
(74) Mandataire: Le Coupanec, Pascale A.M.P.

(57) **Abrégé**

La présente invention concerne une composition cosmétique non compactée de soin et/ou de maquillage des matières kératiniques comprenant, dans un milieu physiologiquement acceptable, au moins 22 % en poids d'eau par rapport au poids total de la composition et au moins une phase grasse comprenant au moins une dispersion de particules de cire micronisée, lesdites particules étant présentes en une teneur comprise entre 5 et 50 % en poids par rapport au poids total de la composition.

## Description

La présente invention concerne une composition cosmétique pour le maquillage et/ou le soin des matières kératiniques, notamment du visage, par exemple de la peau et/ou des lèvres.

Plus particulièrement, les compositions considérées selon l'invention peuvent constituer un produit de maquillage, par exemple du visage, permettant d'obtenir un effet matifiant sur la peau tout en lui préservant un aspect naturel.

Par définition, un produit matifiant est un produit qui empêche la peau de briller.

Des compositions présentant un effet matifiant sont déjà connues. Toutefois, celles-ci ne donnent pas totalement satisfaction.

Les produits de maquillage et/ou de soin de la peau et/ou des lèvres des êtres humains comme les fonds de teint ou rouges à lèvres contiennent généralement une phase grasse à base d'huile(s) et/ou cire(s), des pigments et/ou des charges et éventuellement des additifs comme des actifs cosmétiques ou dermatologiques.

Il est ainsi connu d'utiliser des charges pour obtenir un effet de matité.

Ces charges sont le plus souvent choisies en fonction de leurs bonnes propriétés d'absorption des huiles et/ou de leur capacité à diffuser la lumière de manière efficace.

Les charges peuvent toutefois présenter l'inconvénient de conférer aux compositions dans lesquelles elles sont formulées un aspect poudreux préjudiciable à un aspect naturel, après application de ladite composition sur un support.

Par ailleurs, de telles compositions peuvent présenter une texture lourde et sèche préjudiciable au niveau du toucher et de l'application.

Il existe donc un besoin pour une composition cosmétique fluide et agréable à appliquer permettant d'obtenir un maquillage et/ou un soin matifiant ne présentant pas d'effet poudreux après application.

D'une manière inattendue, les inventeurs ont constaté qu'il était possible de remédier à l'inconvénient précité en incorporant, dans la phase grasse d'une composition de soin et/ou de maquillage, une quantité efficace de particules de cire sous forme micronisée tout en maintenant une teneur minimale en eau suffisante pour assurer de bonnes propriétés cosmétiques.

En conséquence, la présente invention concerne selon un de ses aspects, une composition cosmétique non compactée de soin et/ou de maquillage des matières kératiniques comprenant, dans un milieu physiologiquement acceptable, au moins 22 % en poids d'eau par rapport au poids total de la composition et au moins une phase grasse comprenant au moins une dispersion de particules de cire micronisée, lesdites particules étant présentes en une teneur comprise entre 5 et 50 % en poids par rapport au poids total de la composition.

De préférence, les compositions selon l'invention se présentent sous une forme fluide.

Cette composition peut se présenter notamment sous forme de pâte ou de crème fluide comme les fonds de teint, les bases de maquillage de la peau ou les rouges à lèvres fluides, les eye-liners, les compositions de protection solaire ou de coloration de la peau ou encore de maquillage du corps.

Selon un mode de réalisation particulier, les compositions selon l'invention se présentent sous la forme d'un fond de teint fluide ou d'une base de maquillage de la peau fluide.

Au sens de la présente invention, « fluide » désigne une composition capable de s'écouler sous l'action de son propre poids, à température ambiante (25 °C), par opposition à des composés de type pâte.

D'une manière générale, les compositions selon l'invention possèdent un milieu physiologiquement acceptable.

Par "matières kératiniques", on entend couvrir la peau, les muqueuses, comme les lèvres, les ongles et les fibres kératiniques, à l'image des cils et des cheveux. Les compositions cosmétiques conformes à la présente invention sont particulièrement avantageuses pour une application sur la peau et/ou les lèvres.

Les termes « compris entre ... et ... » et « allant de ... à ... » signifient que les bornes sont également décrites.

### PARTICULES DE CIRE MICRONISEE

De manière générale, le terme « cire » qualifie un composé lipophile, solide à température ambiante (25 °C), à changement d'état solide/liquide réversible, ayant un point de fusion supérieur ou égal à 60 °C et pouvant aller jusqu'à 160 °C, voire 200 °C.

Les particules de cire constituant une des caractéristiques de la présente invention sont sous une forme micronisée.

Par « cire micronisée » ou « microcire », on entend au sens de la présente invention une cire sous la forme d'une poudre dont les particules présentent une taille moyenne en nombre inférieure ou égale à 50 µm, et notamment allant de 0,5 à 50 µm, de préférence allant de 1 à 30 µm, voire allant de 3 à 20 µm.

Par « taille moyenne en nombre », on désigne la dimension donnée par la distribution granulométrique statistique à la moitié de la population, dite D50.

Les microcires pouvant être utilisées dans les compositions selon l'invention sont choisies parmi les cires solides et rigides à température ambiante, d'origine animale, végétale, minérale ou de synthèse et leurs mélanges.

De préférence, les microcires selon l'invention présentent un point de fusion allant de 60 °C à 160 °C, et en particulier supérieur ou égal à 80 °C, notamment allant de 80 °C à 150 °C, voire même supérieur ou égal à 100 °C, notamment allant de 100 °C à 140 °C.

Le point de fusion de la microcire peut être mesuré à l'aide d'un calorimètre à balayage différentiel (D.S.C.), par exemple le calorimètre vendu sous la dénomination DSC 30 par la société METTLER. Un échantillon de 15 mg de produit disposé dans un creuset est soumis à une première montée en température allant de 0 °C à 160 °C, à la vitesse de chauffe de 10 °C/minute, puis est refroidi de 160 °C à 0 °C à une vitesse de refroidissement de 10 °C/minute et enfin soumis à une deuxième montée en température allant de 0 °C à 160 °C à vitesse de chauffe de 5 °C/minute. Pendant, la deuxième montée en température, on mesure la variation de la différence de puissance absorbée par le creuset vide et par le creuset contenant l'échantillon de produit en fonction de la température. Le point de fusion du composé est la valeur de la température correspondant au sommet du pic de la courbe représentant la variation de la différence de puissance absorbée en fonction de la température.

A noter que l'on veillera, dans le procédé de fabrication, à ne pas fondre la microcire. Avantageusement, la température de la composition durant le procédé de fabrication ne dépasse pas une température inférieure à 30 °C par rapport à la température de fusion de la microcire.

Les cires micronisées utilisées dans la présente demande peuvent être choisies parmi les cires micronisées hydrocarbonées, siliconées et/ou fluorées, comportant éventuellement des fonctions ester, amide, acide ou hydroxyle.

Elles sont notamment d'origine naturelle et peuvent être choisies parmi la cire d'abeilles éventuellement modifiée, la cire de Carnauba, de Candelilla, les cires de paraffine, les cires microcristallines, la cire de Montan, les huiles hydrogénées comme l'huile de jojoba hydrogénée ou l'huile de ricin hydrogénée ou résultant de la polymérisation de l'éthylène et/ou du propylène, les cires obtenues par synthèse de Fischer-Tropsch, les esters et les amides d'acides gras, les cires de silicone comme les alkyl-, alcoxy- et/ou esters de poly(di)méthylsiloxane solides à température ambiante, ayant de 10 à 45 atomes de carbone, des acides gras comme l'acide stéarique ou béhénique, et leurs mélanges.

Comme microcires pouvant être utilisées dans les compositions selon l'invention, on peut notamment citer les microcires de Carnauba telles que celles commercialisées sous la dénomination de « MicroCare 350^{®} » par la société MICRO POWDERS, les microcires de cire synthétique telles que celles commercialisées sous la dénomination de « MicroEase 114S^{®} » par la société MICRO POWDERS, les microcires constituées d'un mélange de cire de Carnauba et de cire de polyéthylène telles que celles commercialisées sous les dénominations de « MicroCare 300^{®} » et « 310^{®} » par la société MICRO POWDERS, les microcires constituées d'un mélange de cire de Carnauba et de cire synthétique telles que celles commercialisées sous la dénomination de « MicroCare 325^{®} » par la société MICRO POWDERS, les microcires de polyéthylène telles que celles commercialisées sous les dénominations de « MicroPoly 200^{®} », « 220^{®} », « 220L^{®} », et « 2505^{®} », par la société MICRO POWDERS, et celles commercialisées sous la dénomination de « CERAPURE H5-C » par la société SHAMROCK, ou les microcires de polypropylène telle que celles commercialisées sous la dénomination « MATTEWAX » par la société MICRO POWDERS.

Les particules de cire micronisée peuvent être présentes dans une composition selon l'invention en une teneur comprise entre 5 et 50 % en poids, notamment entre 10 et 30 % en poids, voire entre 15 et 25 % en poids, par rapport au poids total de la composition.

### PHASE AQUEUSE

La composition selon l'invention comprend également au moins un milieu aqueux, constituant une phase aqueuse, qui peut former la phase dispersée ou la phase continue de la composition.

La phase aqueuse peut être constituée exclusivement d'eau.

L'eau peut être une eau florale telle que l'eau de bleuet et/ou une eau minérale telle que l'eau de VITTEL, l'eau de LUCAS ou l'eau de LA ROCHE POSAY et/ou une eau thermale.

La phase aqueuse peut également comprendre un mélange d'eau et de solvant(s) organique(s) miscible(s) à l'eau (miscibilité à l'eau supérieure à 50 % en poids à 25 °C) comme par exemple les monoalcools ayant de 1 à 5 atomes de carbone tels que l'éthanol et l'isopropanol, les glycols ayant de 2 à 8 atomes de carbone tels que le glycérol, le propylène glycol, le 1,3-butylène glycol, le dipropylène glycol, le diéthylène glycol, les éthers de glycol tel que les alkyl(C₁-C₄)éther de mono-, di- ou tri-propylène glycol, mono-, di- ou tri-éthylène glycol, les cétones en C₃-C₄ et les aldéhydes en C₂-C₄, et leurs mélanges.

La phase aqueuse (eau et éventuellement le(s) solvant(s) organique(s) miscible(s) à l'eau) peut comprendre en outre des agents de stabilisation, par exemple le chlorure de sodium, le dichlorure de magnésium et le sulfate de magnésium.

La phase aqueuse peut également comprendre tout composé hydrosoluble ou hydrodispersible compatible avec une phase aqueuse tels que des actifs de soin, des gélifiants, des polymères filmogènes, des épaississants, des tensio-actifs et leurs mélanges.

Les compositions selon l'invention comprennent au moins 22 % en poids d'eau par rapport au poids total de la composition. Elles peuvent comprendre de préférence au moins 25 % en poids d'eau par rapport au poids total de la composition.

La phase aqueuse, et notamment l'eau, peut être présente dans la composition selon l'invention en une teneur allant de 30 à 80 % en poids, et en particulier de 35 à 50 % en poids, par rapport au poids total de la composition.

Comme précisé précédemment, les compositions se présentent avantageusement sous une forme fluide, c'est-à-dire différentes des formes solides de type stick, ou compactées.

La composition se présente avantageusement sous la forme d'une émulsion pouvant être simple, du type huile-dans-eau (H/E) ou eau-dans-huile (E/H) ou d'une émulsion multiple (par exemple eau-huile-eau ou huile-eau-huile) dont la mise en oeuvre est bien connue de l'homme de l'art.

Selon un mode de réalisation particulier de l'invention, la composition se présente sous la forme d'une émulsion eau-dans-huile.

Elle peut également comprendre un tensioactif ou un mélange de tensioactifs dont le HLB (balance hydrophile/lipophile) est généralement adapté à la nature de l'émulsion à stabiliser.

Comme tensioactif utilisable dans l'invention, adapté à l'obtention d'une émulsion E/H on peut citer ceux ayant un HLB inférieur 7 et notamment les ester d'acides gras de polyols comme les mono-, di-, tri- ou sesqui-oléates ou stéarates de sorbitol ou de glycérol, les laurates de glycérol ou de polyéthylène glycol ; les alkyl ou alkoxy diméthicones copolyols à chaîne alkyle ou alkoxy pendante ou en bout de squelette siliconé ayant par exemple de 6 à 22 atomes de carbone. Comme tensioactif utilisable dans l'invention pour l'obtention d'une émulsion H/E on peut citer ceux ayant un HLB supérieur à 7 comme les esters d'acides gras de polyéthylène glycol (monostéarate ou monolaurate de polyéthylène glycol) ; les esters d'acides gras (stéarate, oléate) de sorbitol polyoxyéthylénés ; les alkyl (lauryl, cétyl, stéaryl, octyl) éthers polyoxyéthylénés et les diméthicones copolyols. De façon générale, on peut utiliser tout tensioactif ionique (cationique ou anionique) amphotère et tout tensioactif non ionique, bien connus de l'homme du métier.

Le tensioactif peut être présent dans la composition en une teneur allant de 0,3 à 10 % en poids, et notamment de 1 à 5 % en poids par rapport au poids total de la composition.

### PHASE GRASSE

Les compositions cosmétiques conformes à la présente invention comprennent également une phase grasse comprenant, outre les microcires considérées selon l'invention, notamment au moins un composé choisi parmi les huiles et les corps gras solides à température ambiante (20 - 25 °C) et pression atmosphérique, à l'image par exemple des cires non micronisées et des corps gras pâteux et leurs mélanges.

La phase grasse peut être avantageusement présente en une proportion relative suffisante pour permettre aux particules de cire micronisée considérées selon l'invention d'être effectivement dispersées dans celle-ci.

Ainsi, la phase grasse peut être généralement présente en une teneur totale en poids supérieure ou égale à la teneur totale en poids en particules de cire micronisée.

En particulier, la teneur totale en poids en huiles peut être supérieure ou égale à la teneur totale en poids en particules de cire micronisée.

### a) Huile

On entend par huile, tout corps gras sous forme liquide à température ambiante (20 - 25 °C) et à pression atmosphérique. La phase grasse liquide peut également contenir, outre des huiles, d'autres composés solubilisés dans les huiles tels que des agents gélifiants et/ou structurants.

La composition cosmétique selon la présente invention peut comprendre au moins une, et en particulier au moins deux huiles.

Les huiles convenant à la préparation des compositions cosmétiques selon l'invention peuvent être des huiles volatiles ou non.

Au sens de la présente invention, on entend par "huile volatile", une huile (ou milieu non aqueux) susceptible de s'évaporer au contact de la peau en moins d'une heure, à température ambiante et à pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm Hg), et de préférence allant de 1,3 Pa à 13 000 Pa (0,01 à 100 mm Hg), et préférentiellement allant de 1,3 Pa à 1300 Pa (0,01 à 10 mm Hg).

Au sens de la présente invention, on entend par "huile non-volatile", une huile ayant une pression de vapeur inférieure à 0,13 Pa.

Les huiles volatiles ou non volatiles peuvent être des huiles hydrocarbonées notamment d'origine animale, végétale ou minérale, des huiles synthétiques, des huiles siliconées, des huiles fluorées, ou leurs mélanges.

Au sens de la présente invention, on entend par "huile hydrocarbonée", une huile à squelette hydrocarbonée c'est-à-dire contenant principalement des atomes d'hydrogène et de carbone et éventuellement des atomes d'oxygène, d'azote, de soufre et/ou de phosphore.

Comme huile hydrocarbonée non volatile, on peut notamment citer :
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam^{®}, le squalance^{®} et leurs mélanges, et en particulier le polyisobutène hydrogéné, et
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10.

Les esters peuvent être notamment choisis parmi les esters, notamment d'acide gras comme par exemple :
- l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate ou l'isostéarate d'isopropyle, l'isostéarate d'isostéaryle, le stéarate d'octyle, les esters hydroxylés comme le lactacte d'isostéaryle, l'hydroxystéarate d'octyle, l'adipate de diisopropyle, les heptanoates, et notamment l'heptanoate d'isostéaryle, octanoates, décanoates ou ricinoléates d'alcools ou de polyalcools comme le dioctanoate de propylène glycol, l'octanoate de cétyle, l'octanoate de tridécyle, le 4-diheptanoate et le palmitate d'éthyle 2-hexyle, le benzoate d'alkyle, le diheptanoate de polyéthylène glycol, le diétyl 2-d'hexanoate de propylèneglycol et leurs mélanges, les benzoates d'alcools en C₁₂ à C₁₅, le laurate d'hexyle, les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'isotridécyle, l'isononanoate d'octyle, les esters hydroxylés comme le lactate d'isostéaryle, le malate de di-isostéaryle ;
- les esters de polyols et les esters du pentaérythritol, comme le tétrahydroxystéarate/tétraisostéarate de dipentaérythritol,
- les esters de dimères diols et dimères diacides tels que les Lusplan DD-DA5^{®} et Lusplan DD-DA7^{®}, commercialisés par la société NIPPON FINE CHEMICAL et décrits dans la demande FR0302809 déposée le 6 mars 2003.
- les alcools gras liquides à température ambiante à chaîne carbonée ramifiée et/ou insaturée ayant de 12 à 26 atomes de carbone comme le 2-octyldodécanol, l'alcool isostéarylique, l'alcool oléique, le 2-hexyldécanol, le 2-butyloctanol, et le 2-undécylpentadécanol, et
- les carbonates de di-alkyle, les 2 chaînes alkyles pouvant être identiques ou différentes, tel que le dicaprylyl carbonate commercialisé sous la dénomination CETIOL CC^{®}, par COGNIS.

Les huiles hydrocarbonées volatiles peuvent être choisies parmi les huiles hydrocarbonées ayant de 8 à 16 atomes de carbone, et notamment les alcanes ramifiés en C₈-C₁₆ (appelées aussi isoparaffines) comme l'isododécane (encore appelé 2,2,4,4,6-pentaméthylheptane), l'isodécane, l'isohexadécane, et par exemple les huiles vendues sous les noms commerciaux d'ISOPARS^{®} ou de PERMETHYLS^{®}.

Les compositions selon l'invention peuvent en outre contenir des huiles siliconées volatiles ou non volatiles.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, des groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates, les diméthicones ou phényltriméthicone de viscosité inférieure ou égale à 100 cSt, et leurs mélanges.

Comme huiles de silicones volatiles, on peut plus particulièrement utilisées les huiles de silicones linéaires ou cycliques volatiles, notamment celles ayant une viscosité ≤ 8 centistokes (8 x 10⁻⁶ m²/s), et ayant notamment de 2 à 10 atomes de silicium, et en particulier de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

On peut également utiliser des huiles volatiles fluorées tels que le nonafluorométhoxybutane ou le perfluorométhylcyclopentane, et leurs mélanges.

Une composition selon l'invention peut comprendre en outre une ou plusieurs huile(s) en une teneur allant de 10 à 70 %, en particulier de 12 à 60 %, voire de 15 à 50 % en poids par rapport au poids total de la composition.

Les compositions selon l'invention peuvent également comprendre avantageusement au moins un composé choisi parmi les cires non micronisées, les corps gras pâteux, et leurs mélanges.

### b) Cires non micronisées

Outre les cires micronisées, les compositions selon l'invention peuvent comprendre une ou plusieurs cires choisies, par exemple, parmi, les cires synthétiques comme les cires de polyéthylène (de préférence de poids moléculaire compris entre 400 et 600) ou de Fischer-Tropsch, les cires de silicone comme les alkyl- ou alkoxy-diméthicone ayant de 16 à 45 atomes de carbone, les cires de paraffine, les cérésines, comme par exemple l'EMW-0003, commercialisé par la société NIPPON SEIROU, les oligomères d'α-oléfine, tel que les polymères PERFORMA V^{®} 825, 103 et 260, commercialisés par la société NEW PHASE TECHNOLOGIES ; les copolymères éthylène-propylène, tel que le PERFORMALENE^{®} EP 700, et leurs mélanges.

Une composition selon l'invention peut comprendre une ou plusieurs cire(s) non micronisée(s) en une teneur allant de 0,1 à 30 %, en particulier de 0,2 à 25 %, voire de 0,3 à 20 % en poids, par rapport au poids total de la composition.

### c) Composés pâteux

Les compositions cosmétiques conformes à la présente invention peuvent également comprendre au moins un composé pâteux.

Par "pâteux" au sens de la présente invention, on entend un composé gras à changement d'état solide/liquide réversible et comportant à la température de 25 °C une fraction liquide et une fraction solide.

Conviennent tout particulièrement, à titre de composés pâteux selon l'invention des esters de polyol.

Les esters de polyol utilisables dans le cadre de la présente invention sont disponibles commercialement ou peuvent être préparés de manière conventionnelle. Ils sont généralement d'origine végétale et peuvent notamment être obtenus par mono- ou pluri-estérification d'un polyol, avec un acide mono-carboxylique en C₂-C₃₄ comme par exemple un acide gras ou avec un acide dicarboxylique tel qu'un dimère diacide.

L'ester obtenu peut être, notamment, un polyester, un triester, un diester, un monoester ou un de leurs mélanges. En l'occurrence, l'ester peut être un mélange de deux ou plusieurs types d'ester formés avec différents acides carboxyliques.

Dans le cas de l'estérification avec un acide mono-carboxylique, on peut obtenir des esters ayant un poids moléculaire relativement élevé, allant d'environ 200 à 1300 g/mol.

Dans la réaction d'estérification avec un acide di-carboxylique, on peut obtenir un di-carboxylate de polyol qui présente un poids moléculaire moyen en poids, déterminé par chromatographie de perméation de gel (GPC), allant de 200 à 20 000 g/mol, de préférence entre 2000 et 4000 g/mol.

Par "polyol" ou "alcool polyhydrique", il faut comprendre, au sens de la présente invention, toute molécule organique comportant au moins deux groupements hydroxyle libres.

En particulier, ils peuvent posséder de 2 à 20 atomes de carbone, en particulier de 3 à 10 atomes de carbone, et plus particulièrement de 4 à 6 atomes de carbone.

Avantageusement, le polyol peut être par exemple choisi parmi un dimère diol, la glycérine, le propylène glycol, le butylène glycol, le pentylène glycol, l'hexylène glycol, le dipropylène glycol, le diéthylène glycol, le sorbitol, l'hydroxypropyl sorbitol, le 1,2,6-hexanetriol ;les éthers de glycol (ayant notamment de 3 à 16 atomes de carbone) tels que les alkyl(C₁-C₄) éther de mono-, di- ou tri-propylène glycol, les alkyl(C₁-C₄)éthers de mono-, di- ou tri-éthylène glycol ; et leurs mélanges.

Il peut également s'agir de "dimère diol", c'est-à-dire des diols saturés produits par hydrogénation des dimères diacides correspondants.

Un dimère diol peut être produit par hydrogénation d'un dimère diacide, lui-même obtenu par dimérisation d'un acide gras insaturé notamment en C₈ à C₃₄, tels que ceux cités précédemment, notamment en C₁₂ à C₂₂ et en particulier en C₁₆ à C₂₀, de préférence en C₁₈ à l'image par exemple de l'acide oléique et de l'acide linoléique.

Les polyols convenant plus particulièrement sont des sucres choisis parmi les monosaccharides, disaccharides et trisaccharides. A titre représentatif de ces sucres, on peut notamment citer les monosaccharides tels que le xylose, l'arabinose, le galactose, le fructose, le mannose, le glucose et leurs mélanges. A titre représentatif des polyols disaccharides, on peut plus particulièrement citer le maltose, le lactose, le saccharose et leurs combinaisons.

L'acide mono-carboxylique utilisable dans la présente invention peut comporter de 2 à 34 atomes de carbone, et notamment de 10 à 32 atomes de carbone.

A titre illustratif des exemples d'acide mono-carboxylique convenant à l'invention, on peut notamment citer :
- les acides linéaires saturés tels que l'acide butanoïque, l'acide pentanoïque, l'acide hexanoïque, l'acide heptanoïque, l'acide octanoïque, l'acide nonanoïque, l'acide décanoïque, l'acide undécanoïque, l'acide dodécanoïque, l'acide tridécanoïque, l'acide tétradécanoïque, l'acide pentadécanoïque, l'acide hexadécanoïque, l'acide heptadécanoïque, l'acide octadécanoïque, l'acide nonadécanoïque, l'acide eicosanoïque, l'acide docosanoïque, l'acide tétracosanoïque,
- les acides gras ramifiés tels que par exemple l'acide isobutanoïque, l'acide isopentanoïque, l'acide pivalique, l'acide isohexanoïque, l'acide isoheptanoïque, l'acide isooctanoïque, l'acide diméthyloctanoïque, l'acide isononanoïque, l'acide isodécanoïque, l'acide isoundécanoïque, l'acide isododécanoïque, l'acide isotridécanoïque, l'acide isotétradécanoïque, l'acide isopentadécanoïque, l'acide isohexadécanoïque, l'acide isoheptadécanoïque, l'acide isooctadécanoïque, l'acide isononadécanoïque, l'acide isoeicosanoïque, l'acide 2-éthylhexanoïque, l'acide 2-butyloctanoïque, l'acide 2-hexyldécanoïque, l'acide 2-octyldodécanoïque, l'acide 2-décyltétradécanoïque, l'acide 2-dodécylhexadécanoïque, l'acide 2-tétradécyloctadécanoïque, l'acide 2-hexadécyloctadécanoïque, des acides gras à longue chaîne obtenus à partir de la lanoline,
- les acides gras linéaires insaturés en C₈ à C₃₄, tels que l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique,
- les hydroxyacides tels que l'acide 2-hydroxybutanoïque, l'acide 2-hydropentanoïque, l'acide 2-hydroxyhexanoïque, l'acide 2-hydroxyheptanoïque, l'acide 2-hydroxyoctanoïque, l'acide 2-hydroxynonanoïque, l'acide 2-hydroxydécanoïque, l'acide 2-hydroxyundécanoïque, l'acide 2-hydroxydodécanoïque, l'acide 2-hydroxytridécanoïque, l'acide 2-hydroxytétradécanoïque, l'acide 2-hydroxyhexadécanoïque, l'acide 2-hydroxyheptadécanoïque, l'acide 2-hydroxyoctadécanoïque, l'acide 12-hydroxyoctadécanoïque, l'acide 2-hydroxynonadécanoïque, l'acide 2-hydroxyeicosanoïque, l'acide 2-hydroxydocosanoïque, l'acide 2-hydroxytétracosanoïque,
- les acides cycliques tels que l'acide cyclohexanoïque, la rosine hydrogénée, la rosine, l'acide abiétique, l'acide abiétique hydrogéné, l'acide benzoïque, l'acide p-oxybenzoïque, l'acide p-aminobenzoïque, l'acide cinnamique, l'acide p-méthoxycinnamique, l'acide salicylique, l'acide gallique, l'acide pyrrolidonecarboxylique, l'acide nicotinique, et
- les acides gras d'origine naturelle, tels que les acides gras d'huile d'orange, d'huile d'avocat, d'huile de macadamia, d'huile d'olive, d'huile de soja hydrogénée, d'huile de jojoba, d'huile de palme, d'huile de ricin, d'huile de germe de blé, d'huile de safran, d'huile de grains de coton, d'huile de vison et leurs mélanges.

Il s'agit plus particulièrement d'un acide gras, notamment tel que défini ci-dessus.

L'acide dicarboxylique utilisable selon l'invention peut contenir au moins deux groupes carboxyliques par molécule.

Il peut notamment être représenté par la formule suivante :

HOOC-(CH₂)ₙ-COOH

dans laquelle n est un nombre entier de 1 à 16, de préférence de 3 à 16.

A titre illustratif et non limitatif des acides dicarboxyliques convenant à l'invention, on peut notamment citer l'acide malonique, l'acide succinique, l'acide glutarique, l'acide adipique, l'acide pimélique, l'acide subérique, l'acide azélaïque, l'acide sébacique, l'acide 1,9-nonaméthylène-dicarboxylique, l'acide 1,10-décaméthylènedicarboxylique, l'acide 1,11-undécaméthylènedicarboxylique, l'acide 1,12-dodécaméthylène-dicarboxylique, l'acide 1,13-tridécaméthylènedicarboxylique, l'acide 1,14-tétradécaméthylènedicarboxylique, l'acide 1,15-pentadécaméthylènedicarboxylique, l'acide 1,16-hexadécaméthylènedicarboxylique et leurs mélanges.

L'acide dicarboxylique peut également être un dimère diacide. Un dimère diacide désigne un diacide obtenu par réaction de polymérisation, notamment de dimérisation, intermoléculaire d'au moins un acide gras insaturé notamment en C₈ à C₃₄, tels que ceux cités précédemment, notamment en C₁₂ à C₂₂, en particulier en C₁₆ à C₂₀, de préférence en C₁₈ à l'image par exemple de l'acide oléique et de l'acide linoléique.

Conviennent également tout particulièrement à titre d'esters de polyol, les polyesters de polyol dans lesquels les motifs esters acide gras du polyester comprennent des longueurs de chaînes saturées ou insaturées choisies de manière à ce que le composé possède le comportement requis en terme de composés pâteux selon l'invention.

Les chaînes d'acide gras insaturées sont typiquement des chaînes ramifiées et contiennent plus particulièrement de 12 à environ 22, et plus particulièrement d'environ 18 à 22 atomes de carbone.

Les chaînes d'acide gras insaturées plus particulièrement considérées sont les acides gras mono- et/ou di- insaturées en C₁₈.

A ces longues chaînes peuvent être associées des chaînes plus courtes d'acides gras saturés. Elles sont généralement linéaires et contiennent de 2 à environ 12, de préférence de 6 à environ 12, et plus particulièrement de 8 à 12 atomes de carbone.

De manière générale, le taux d'estérification de ces esters d'acides gras est tel qu'environ 60 % des fonctions hydroxyles des polyols sont estérifiées, et plus particulièrement environ 85 %, voire 95 % des fonctions hydroxyles.

En ce qui concerne les motifs esters d'acides gras à longues chaînes insaturées, on peut plus particulièrement citer les lauroléates, myristoléates, palmitoléates, oléates, élaïdates, éructates, linoléates, linolénates, arachidonates, éicosapentaénoates et docosahexaénoates. Pour des raisons de stabilité à l'oxydation, les chaînes d'acides gras mono- et di-insaturées sont préférées.

En ce qui concerne les motifs esters acides gras insaturés à longues chaînes saturées, on peut particulièrement citer les esters arachidates, béhénates, linosérates et sérotates.

En ce qui concerne les motifs esters acides gras saturés à chaînes courtes, il s'agit plus particulièrement de l'acétate, caproate, caprilate, caprate et laurate.

A titre de polyesters d'acide gras polyol solide convenant tout particulièrement à l'invention, on peut plus particulièrement citer les octa-esters de raffinose dans lesquels les parties acides gras estérifiantes sont le linoléate et le béhénate, les hecta esters de maltose dans lesquels les parties acides gras estérifiantes dérivent de l'acide gras d'huile de graine de tournesol et de lignosérate, les octa-esters de saccharose dans lesquels les parties acides gras estérifiantes sont le béhénate et l'oléate et les octa-esters de saccharose dans lesquels les parties acide gras estérifiantes sont les laurates, linoléates et béhénates.

De tels polyesters d'acides gras solides peuvent être obtenus selon des méthodes déjà décrites pour la préparation des polyesters de polyols. A ce titre, on peut notamment se référer aux documents US 5 306 516, US 5 306 515, US 5 305 514, US 4 797 300, US 3 963 699, US 4 518 772 et US 4 517 360.

Comme composé pâteux convenant avantageusement à la formulation des compositions cosmétiques conformes à la présente invention, on peut plus particulièrement faire mention des triglycérides hydrogénés fractionnés et notamment ceux commercialisés par SIO ; des huiles végétales hydrogénées, de l'huile de palme hydrogénée du beurre de cacao et par exemple celles commercialisées par KARLSHAMNS, de l'huile de graine de coton solide et par exemple celle commercialisée par SIO, du sucrose acétate isobutyrate et par exemple celui commercialisé par EASTMAN CHEMICAL.

Parmi les autres composés pâteux susceptibles d'être utilisés dans la composition selon l'invention, on peut également citer les lanolines et les dérivés de lanoline comme les lanolines acétylées, les lanolines oxypropylénées ou le lanolate d'isopropyle, et leurs mélanges.

On peut aussi citer les composés pâteux siliconés tels que les polydiméthylsiloxanes (PDMS) de hauts poids moléculaires et en particulier ceux ayant des chaînes pendantes du type alkyle ou alcoxy ayant de 8 à 24 atomes de carbone, et un point de fusion de 20-55°C, comme les stéaryl diméthicones notamment ceux vendus par la société DOW CORNING sous les noms commerciaux de DC2503^{®} et DC25514^{®} et leurs mélanges.

Une composition selon l'invention peut comprendre un ou plusieurs composé(s) pâteux en une teneur allant de 0,1 à 50 %, en particulier de 0,2 à 40 %, voire de 0,3 à 30 % en poids par rapport au poids total de la composition.

La composition selon l'invention peut comprendre en outre au moins un composé pulvérulent annexe, c'est-à-dire différent des microcires considérées selon l'invention. Le composé pulvérulent annexe peut être choisi parmi les charges, les matières colorantes pulvérulentes, telles que les pigments et les nacres, et leurs mélanges.

Par « charges », il faut comprendre des particules incolores ou blanches, minérales ou de synthèse, lamellaires ou non lamellaires.

Ces charges peuvent être choisies parmi le talc, le mica, la silice, le kaolin, les poudres de poly-β-alanine, la lauroyl-lysine, l'amidon, le nitrure de bore, les microsphères creuses polymériques telles que celles de chlorure de polyvinylidène/acrylonitrile telles que l'Expancel^{®} (Nobel Industrie), les particules de polymère acrylique, notamment de copolymère d'acide acrylique comme le Polytrap^{®} (Dow Corning), les poudres de polyuréthane, les poudres de nylon, les microbilles de résine de silicone (Tospearls^{®} de Toshiba, par exemple), le carbonate de calcium précipité, le dicalcium phosphate, le carbonate et l'hydro-carbonate de magnésium, l'hydroxyapatite, les microsphères de silice creuses (Silica Beads^{®} de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium et leurs mélanges.

Ces charges peuvent être traitées ou non en surface notamment pour les rendre lipophiles.

Ces charges peuvent être présentes dans la composition selon l'invention en une teneur allant de 0,1 à 30 % en poids, et notamment de 0,1 à 15 % en poids par rapport au poids total de la composition.

Une composition selon l'invention peut comprendre en outre au moins une matière colorante choisie parmi les matières colorantes pulvérulentes, telles que les pigments et les nacres, les colorants tels que les colorants lipophiles et les colorants hydrophiles, et leurs mélanges.

Par « pigments », on entend désigner, au sens de la présente invention, des particules blanches ou colorées, minérales ou organiques, insolubles dans la phase organique liquide, destinées à colorer et/ou opacifier la composition.

Les pigments peuvent être des pigments minéraux et/ou organiques. Comme pigments, on peut utiliser les oxydes métalliques comme les oxydes de fer (notamment ceux de couleur jaune, rouge, brun, noir), les dioxydes de titane, l'oxyde de cérium, l'oxyde de zirconium, l'oxyde de chrome ; le violet de manganèse, l'ultramarine bleue, le bleu de prusse, le bleu outremer, le bleu ferrique, l'oxychlorure de bismuth, la nacre, le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth, et leurs mélanges.

On utilise en particulier des pigments d'oxydes de fer ou de dioxyde de titane.

Les matières colorantes pulvérulentes, et notamment les pigments, peuvent être traités en surface, le cas échéant, avec un agent hydrophobe pour les rendre compatible avec la phase grasse de la composition.

De tels pigments traités hydrophobes sont notamment décrits dans la demande EP-A-1086683.

A titre illustratif d'agent hydrophobe pouvant être utilisé pour enrober des pigments, on peut par exemple citer le stéaroyl glutamate d'aluminium, et de préférence le tri-isostéaroyl titanate d'isopropyle.

Par « nacres », on entend désigner, au sens de la présente invention, des particules irisées, notamment produites par certains mollusques dans leur coquille ou bien synthétisées, insolubles dans le milieu de la composition.

Par « colorant », on entend désigner, au sens de la présente invention, des composés généralement organiques solubles dans les corps gras comme les huiles ou dans une phase aqueuse et/ou hydroalcoolique.

Parmi les colorants liposolubles utilisables selon l'invention, on peut citer le rouge Soudan, le D&C Red n° 17, le D&C Green n° 6, le β-carotène, l'huile de soja, le brun Soudan, le D&C Yellow n° 11, le D&C Violet n° 2, le D&C orange n° 5, le jaune quinoléine, le rocou, et les bromoacides.

Les matières colorantes pulvérulentes peuvent être présents dans une composition selon l'invention en une teneur allant de 0,5 à 30 % en poids, notamment allant de 5 à 20 % en poids, en particulier allant de 8 à 15 % en poids, par rapport au poids total de la composition.

Les colorants lipophiles et/ou hydrophiles peuvent être présents dans une composition selon l'invention en une teneur allant de 0,0001 à 10 % en poids, de préférence de 0,01 à 5 % en poids, et encore plus préférentiellement de 0,1 à 3 % en poids, par rapport au poids total de la composition.

La composition selon l'invention peut contenir en outre un ou plusieurs des adjuvants habituels dans le domaine cosmétique tels que des agents gélifiants et/ou épaississants hydrophiles ou lipophiles ; des agents hydratants ; des émollients ; des actifs hydrophiles ou lipophiles ; des agents anti-radicaux libres ; des séquestrants ; des antioxydants ; des conservateurs ; des agents alcanisants ou acidifiants ; des parfums ; et leurs mélanges.

Comme actifs utilisables dans la composition selon l'invention, on peut citer par exemple les agents hydratants tels que les hydrolysats de protéines et les polyols comme la glycérine, les glycols comme les polyéthylène glycols, et les dérivés de sucre ; les extraits naturels ; les anti-inflammatoires ; les oligomères procyannidoliques ; les vitamines comme la vitamine A (rétinol), la vitamine E (tocophérol), la vitamine C (acide ascorbique), la vitamine B5 (panthénol), la vitamine B3 (niacinamide), les dérivés de ces vitamines (notamment esters) et leurs mélanges ; l'urée ; la caféine ; l'acide salicylique et ses dérivés ; les alpha-hydroxyacides tels que l'acide lactique et l'acide glycolique et leurs dérivés ; les rétinoïdes tels que les caroténoïdes et les dérivés de vitamine A ; l'hydrocortisone ; la mélatonine ; les extraits d'algues, de champignons, de végétaux, de levures, de bactéries ; les enzymes ; les stéroïdes ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les agents tenseurs ; et leurs mélanges.

La composition selon l'invention peut en outre comprendre au moins un filtre solaire (ou filtres U.V.). Celui-ci peut être choisi parmi les filtres organiques, les filtres physiques et leurs mélanges.

Comme filtres solaires chimiques utilisables dans la composition de l'invention, la composition de l'invention peut comprendre tous les filtres UVA et UVB utilisables dans le domaine cosmétique.

Bien entendu, l'homme de l'art veillera à choisir le ou les éventuels adjuvants ajoutés à la composition selon l'invention et à ajuster leurs quantités de manière telle que les propriétés avantageuses attachées intrinsèquement à la composition conforme à l'invention ne soient pas, ou substantiellement pas, altérées par l'adjonction considérée.

Les exemples suivants sont donnés à titre illustratif et sans caractère limitatif de l'invention.

### Exemple 1 : Fond de teint liquide

| | % massique |
|---|---|
| A1 | |
| - Cétyl PEG/PPG-10/1 Diméthicone, vendu sous la référence ABIL EM 90 par la Société Goldschmidt | 2,37 |
| - Polyglycéryl-4 isostéarate, vendu sous la référence ISOLAN GI 34 par la Société Goldschmidt | 0,39 |
| - Butyl parabène | 0,08 |
| - Tristéarine et stéarate d'éthylène glycol acétylé, vendu sous la référence UNITWIX par la Société United Guardian | 0,39 |
| | |

| A2 | |
|---|---|
| - Cyclopentasiloxane et PEG/PPG-18/18 diméthicone, vendu sous la référence DC 5225C FORMULATION AID par la Société DOW CORNING | 2,02 |
| | |

| A3 | |
|---|---|
| - Cyclopentasiloxane, vendu sous la référence DC 245 FLUID par la Société DOW CORNING | 16,90 |
| - Diméthicone, vendue sous la référence DC FLUID 200 5 cSt par la Société DOW CORNING | 8,40 |
| | |

| A4 | |
|---|---|
| - Microcire synthétique, vendue sous la référence MICROEASE 114S par la Société MICRO POWDERS | 16,80 |
| | |

| A5 | |
|---|---|
| - Oxyde de fer jaune enrobé de stéaroyl glutamate d'aluminium, vendu sous la référence NAI-C33 - 8073 - 10 par la Société Myoshi Kasei | 1,55 |
| - Oxyde de fer rouge enrobé de stéaroyl glutamate d'aluminium, vendu sous | |
| la référence NAI - C33 - 8075 - 10 par la Société Myoshi Kasei | 0,32 |
| - Oxyde de fer noir enrobé de stéaroyl glutamate d'aluminium, vendu sous la référence NAI - C33 - 134 - 10 par la Société Myoshi Kasei | 0,16 |
| - Dioxyde de titane enrobé de stéaroyl glutamate d'aluminium, vendu sous la référence NAI - C47 - 051 - 10 par la Société Myoshi Kasei | 8,87 |
| - Oxyde de fer enrobé de stéaroyl glutamate d'aluminium, vendu sous la référence NAI - C33 - 115 - 10 par la Société Myoshi Kasei | 0,10 |
| - Cyclopentasiloxane, vendu sous la référence DC 245 FLUID par la Société DOW CORNING | 5,00 |
| | |

| B1 | |
|---|---|
| - Eau | 28,98 |
| - Méthyl parabène | 0,16 |
| - Butylène glycol | 3,96 |
| - Sulfate de magnésium | 0,55 |
| - Propylène glycol | 2,37 |
| | |

| B2 | |
|---|---|
| - Hydroxypropyl Guar, vendu sous la référence JAGUAR HP 105 par la Société RHODIA | 0,08 |
| | |

| B3 | |
|---|---|
| - Phénoxyéthanol | 0,55 |

### Mode opératoire

Les constituants de la phase A1 sont pesés puis placés au bain-marie (100 °C). Lorsque le mélange est homogène, la phase A2 est ajoutée sous agitation (Moritz à 2000 trs/min) puis la phase A3, à une température de 60 °C, et enfin la phase A4, à une température de 50 °C, en maintenant l'agitation.

La phase A5 est préparée séparément en broyant les pigments à la tri-cylindre puis ajoutée sous agitation au mélange précédent.

La phase aqueuse est préparée en pesant les constituants de la phase B1 portés ensuite à ébullition.

On place alors cette phase sous agitation (Raynerie à une vitesse de 400-500 trs/min) et on y ajoute la phase B2. L'agitation est maintenue jusqu'à l'obtention d'un gel lisse. La phase B3 est alors ajoutée, à une température de l'ordre de 40 °C.

L'émulsion se fait à température ambiante. Les deux phases doivent avoir une température proche (pas plus de 3 °C d'écart) et voisine de la température ambiante.

La phase aqueuse est versée dans la phase grasse en augmentant progressivement la vitesse d'agitation (Moritz jusqu'à 4000-4500 trs/min). Après addition, l'agitation est maintenue durant 10 minutes.

### Résultat

Ce fond de teint s'applique très bien, il a un bon glissant et il est très doux et frais à l'application.

Le maquillage résultant est uniforme, mat, sans effet poudreux. Il a un toucher très doux et il ne colle pas.

### Exemple 2 : Fond de teint liquide

| | % massique |
|---|---|
| A1 | |
| - Cétyl PEG/PPG-10/1 Diméthicone, vendu sous la référence ABIL EM 90 par la Société Goldschmidt | 2,26 |
| - Polyglycéryl-4 isostéarate, vendu sous la référence ISOLAN GI 34 par la Société Godschmidt | 0,38 |
| - Butyl parabène | 0,07 |
| - Tristéarine et stéarate d'éthylène glycol acétylé, vendu sous la référence UNITWIX par la Société United Guardian | 0,38 |
| | |

| A2 | |
|---|---|
| - Cyclopentasiloxane et PEG/PPG-18/18 diméthicone, vendu sous la référence DC 5225C FORMULATION AID par la Société DOW CORNING | 1,93 |

| A3 | |
|---|---|
| - Cyclopentasiloxane, vendu sous la référence DC 245 FLUID par la Société DOW CORNING | 15,93 |
| - Diméthicone, vendue sous la référence DC FLUID 200 5 cSt par la Société DOW CORNING | 8,03 |
| | |

| A4 | |
|---|---|
| - Microcire synthétique, vendue sous la référence MICROEASE 114S par la Société MICROPOWDERS | 20,00 |
| | |

| A5 | |
|---|---|
| - Oxyde de fer jaune enrobé de stéaroyl glutamate d'aluminium, vendu sous la référenceNAI - C33 - 8073 - 10 par la société Myoshi Kasei | 2,70 |
| - Oxyde de fer rouge enrobé de stéaroyl glutamate d'aluminium, vendu sous la référence NAI - C33 - 8075 - 10 par la Société Myoshi Kaséi | 0,52 |
| - Oxyde de fer noir enrobé de stéaroyl glutamate d'aluminium, vendu sous la référenceNAI - C33 - 134 - 10 par la Société Myoshi Kaséi | 0,21 |
| - Dioxyde de titane enrobé de stéaroyl glutamate d'auluminium, vendu sous la référence NAI - C47 - 051 - 10 par la Société Myoshi Kaséi | 7,45 |
| - Oxyde de fer enrobé de stéaroyl glutamate d'aluminium, vendu sous la référence NAI - C33 - 115 - 10 par la Société Myoshi Kaséi | 0,12 |
| - Cyclopentasiloxane, vendu sous la référence (DC 245 FLUID) par la Société DOW CORNING | 5,00 |
| | |

| B1 | |
|---|---|
| - Eau | 27,70 |
| - Méthyl parabène | 0,15 |
| - Butylène glycol | 3,78 |
| - Sulfate de magnésium | 0,53 |
| - Propylène glycol | 2,26 |

| B2 | |
|---|---|
| - Hydroxypropyl Guar, vendu sous la référence JAGUAR HP 105 par la Société Rhodia | 0,07 |
| | |

| B3 | |
|---|---|
| - Phénoxyéthanol | 0,53 |

### Mode opératoire

Le mode opératoire est le même que celui mis en oeuvre dans l'exemple 1.

Ce fond de teint a des propriétés sensorielles très proches de celui de l'exemple 1.

### Exemple 3 : Fond de teint liquide

| | % massique |
|---|---|
| A1 | |
| - Cétyl PEG/PPG-10/1 Diméthicone, vendu sous la référence ABIL EM 90 par la Société Goldschmidt | 2,37 |
| - Polyglycéryl-4 isostéarate, vendu sous la référence ISOLAN GI 34 par la Société Goldschmidt | 0,39 |
| - Butyl parabène | 0,08 |
| - Tristéarine et stéarate d'éthylène glycol acétylé, vendu sous la référence UNITWIX par la Société United Guardian | 0,50 |
| | |

| A2 | |
|---|---|
| - Cyclopentasiloxane et PEG/PPG-18/18 diméthicone, vendu sous la référence DC 5225C FORMULATION AID par la Société DOW CORNING | 2,02 |
| | |

| A3 | |
|---|---|
| - Cyclopentasiloxane, vendu sous la référence DC 245 FLUID par la Société DOW CORNING | 15,79 |
| - Diméthicone, vendue sous la référence DC FLUID 200 5 cSt par la Société DOW CORNING | 8,40 |

| A4 | |
|---|---|
| - Microcire synthétique, vendue sous la référence MICROEASE 114S par la Société MICRO POWDERS | 16,80 |
| | |

| A5 | |
|---|---|
| - Oxyde de fer jaune enrobé de tri-iso-stéaroyl -titanate d'iso-propyle, vendu sous la référence BYO-I 2 par la Société Kobo | 2,13 |
| - Oxyde de fer rouge enrobé de tri-iso-stéaroyl titanate d'iso-propyle, vendu sous la référence BRO-1 2 par la Société Kobo | 0,72 |
| - Oxyde de fer noir enrobé de tri-iso-stéaroyl titanate d'iso-propyle, vendu sous la référence BBO-I 2 par la Société Kobo | 0,29 |
| - Dioxyde de titane enrobé de tri-iso-stéaroyl titanate d'iso-propyle, vendu sous la référence BTD-401 par la Société Kobo | 7,86 |
| - Cyclopentasiloxane DC 245 FLUID par la Société DOW CORNING | 5,00 |
| | |

| B1 | |
|---|---|
| - Eau | 29,98 |
| - Méthyl parabène | 0,16 |
| - Butylène glycol | 3,96 |
| - Sulfate de magnésium | 0,55 |
| - Propylène glycol | 2,37 |
| | |

| B2 | |
|---|---|
| - Hydroxypropyl Guar, vendu sous la référence JAGUAR HP 105 par la Société Rhodia | 0,08 |
| | |

| B3 | |
|---|---|
| - Phénoxyéthanol | 0,55 |

### Mode opératoire

Le mode opératoire est le même que celui mes en oeuvre dans l'exemple 1.

Ce fond de teint a des propriétés sensorielles très proches de celui de l'exemple 1.

### Exemple 4 : Fond de teint liquide

| | % massique |
|---|---|
| A1 | |
| - Cétyl PEG/PPG-10/1 Diméthicone, vendu sous la référence ABIL EM 90 par la Société Goldschmidt | 2,26 |
| - Polyglycéryl-4 isostéarate vendu sous la référence ISOLAN GI 34 par la Société Goldschmidt | 0,38 |
| - Butyl parabène | 0,07 |
| - Tristéarine et stéarate d'éthylène glycol acétylé, vendu sous la référence UNITWIX par la Société United Guardian | 0,38 |
| | |

| A2 | |
|---|---|
| - Cyclopentasiloxane et PEG/PPG-18/18 Diméthicone, vendu sous la référence DC 5225 FORMULATION AID par la Société DOW CORNING | 1,93 |
| | |

| A3 | |
|---|---|
| - Cyclopentasiloxane, vendu sous la référence DC 245 FLUID par la Société DOW CORNING | 15,93 |
| - Diméthicone, vendue sous la référence DC FLUID 200 5 cST par la Société DOW CORNING | 8,03 |
| | |

| A4 | |
|---|---|
| - Microcire de Carnauba, vendue sous la référence MICROCARE 350 par la Société MICRO POWDERS | 20,00 |
| | |

| A5 | |
|---|---|
| - Oxyde de fer jaune enrobé de stéaroyl glutamate d'aluminium, vendu sous la référence NAI-C33-8073-10 par la Société MYOSHI KASEI | 2,7 |
| - Oxyde de fer rouge enrobé de stéaroyl glutamate d'aluminium, vendu sous la référence NAI-C33-8075-10 par la Société MYOSHI KASEI | 0,52 |
| - Oxyde de fer noir enrobé de stéaroyl glutamate d'aluminium, vendu sous la référence NAI-C33-134-10 par la Société MYOSHI KASEI | 0,21 |
| - Dioxyde de titane enrobé de stéaroyl glutamate d'aluminium, vendu sous la référence NAI-C47-051 -10 par la Société MYOSHI KASEI | 7,45 |
| - Oxydes de fer enrobés de stéaroyl glutamate d'aluminium, vendu sous la référence NAI-C33-115-10 par la Société MYOSHI KASEI | 0,12 |
| - Cyclopentasiloxane vendu sous la référence DC 245 FLUID par la société DOW CORNING | 5,00 |
| | |

| B1 | |
|---|---|
| - Eau | 27,70 |
| - Méthyl parabène | 0,15 |
| - Butylène glycol | 3,78 |
| - Sulfate de magnésium | 0,53 |
| - Propylène glycol | 2,26 |
| | |

| B2 | |
|---|---|
| - Hydroxydroxypropyl guar, vendu sous la référence JAGUAR HP 105 par la Société RHODIA | 0,07 |
| | |

| B3 | |
|---|---|
| - Phénoxyéthanol | 0,53 |

### Mode opératoire

Le mode opératoire est le même que celui mis en oeuvre dans l'exemple 1.

Le fond de teint a des propriétés sensorielles très proches de celui de l'exemple 1.

### Exemple 5 : Base de maquillage

| | % massique |
|---|---|
| A1 | |
| - Cétyl PEG/PPG-10/1 Diméthicone, vendu sous la référence ABIL EM 90 par la Société Godschmidt | 3,00 |
| - Polyglycéryl-4 isostéarate, vendu sous la référence ISOLAN GI 34 par la Société Godschmidt | 0,50 |
| | |
| - Butyl parabène | 0,10 |
| | |
| - Tristéarine et stéarate d'éthylène glycol acétylé, vendu sous la référence UNITWIX par la Société United Guardian | 0,50 |
| | |

| A2 | |
|---|---|
| - Cyclopentasiloxane et PEG/PPG-18/18 diméthicone, vendu sous la référence DC 5225C FORMULATION AID par la Société DOW CORNING | 2,55 |
| | |

| A3 | |
|---|---|
| - Cyclopentasiloxane, vendu sous la reference DC 245 FLUID par la Société DOW CORNING | 22,00 |
| - Diméthicone, vendue sous la référence DC FLUID 200 5 cSt par la Société DOW CORNING | 10,00 |
| | |

| A4 | |
|---|---|
| - Microcire synthétique, vendue sous la référence MICROEASE 114S par la Société MICRO POWDERS | 15,00 |
| | |

| B1 | |
|---|---|
| - Eau | 36,65 |
| - Méthyl parabène | 0,20 |
| - Butylène glycol | 5,00 |
| - Sulfate de magnésium | 0,70 |
| - Propylène glycol | 3,00 |
| | |

| B2 | |
|---|---|
| - Hydroxypropyl Guar, vendu sous la référence JAGUAR HP 105 par la Société RHODIA | 0,10 |

| B3 | |
|---|---|
| - Phénoxyéthanol | 0,70 |

### Mode opératoire

Les constituants de la phase A1 sont pesés puis placés au bain-marie (100 °C). Lorsque le mélange est homogène, la phase A2 est ajoutée sous agitation (Moritz à 2000 trs/min) puis la phase A3, à une température de 60 °C, et enfin la phase A4, à une température de 50 °C, en maintenant l'agitation.

La phase aqueuse est préparée en pesant les constituants de la phase B1 portés ensuite à ébullition. On place alors cette phase sous agitation (Raynerie à une vitesse de 400-500 trs/min) et on y ajoute la phase B2. L'agitation est maintenue jusqu'à l'obtention d'un gel lisse. La phase B3 est alors ajoutée, à une température de l'ordre de 40 °C.

L'émulsion se fait à température ambiante. Les deux phases doivent avoir une température proche (pas plus de 3 °C d'écart) et voisine de la température ambiante.

La phase aqueuse est versée dans la phase grasse en augmentant progressivement la vitesse d'agitation (Moritz jusqu'à 4000-4500 trs/min). Après addition, l'agitation est maintenue durant 10 minutes.

### Résultat

Cette base s'applique très bien, elle a un bon glissant et elle est très douce et fraîche à l'application. Le résultat est uniforme, mat, sans effet poudreux. Il a un toucher très doux et il ne colle pas.

Cette base de maquillage peut être appliquée en association avec un fond de teint ou encore une poudre libre ou compactée.

## Revendications

1. Composition cosmétique non compactée de soin et/ou de maquillage des matières kératiniques comprenant, dans un milieu physiologiquement acceptable, au moins 22 % en poids d'eau par rapport au poids total de la composition et au moins une phase grasse comprenant au moins une dispersion de particules de cire micronisée, lesdites particules étant présentes en une teneur comprise entre 5 et 50 % en poids par rapport au poids total de la composition.

2. Composition selon la revendication précédente, comprenant au moins 25 % en poids d'eau par rapport au poids total de la composition.

3. Composition selon la revendication 1 ou 2, dans laquelle les particules de cire micronisée sont présentes en une teneur comprise entre 10 et 30 % en poids, voire entre 15 et 25 % en poids, par rapport au poids total de la composition.

4. Composition selon l'une quelconque des revendications 1 à 3, dans laquelle les particules de cire micronisée présentent une taille moyenne en nombre inférieure ou égale à 50 µm et notamment allant de 0,5 et 50 µm, de préférence de 1 à 30 µm, voire allant de 3 et 20 µm.

5. Composition selon l'une quelconque des revendications précédentes, dans laquelle la cire micronisée est choisie parmi les cires micronisées hydrocarbonées, siliconées et/ou fluorées, comportant éventuellement des fonctions ester, amide, acide ou hydroxyle.

6. Composition selon l'une quelconque des revendications précédentes dans laquelle la cire micronisée est choisie parmi la cire d'abeilles éventuellement modifiée, la cire de Carnauba, de Candelilla, les cires de paraffine, les cires microcristallines, la cire de Montan, les huiles hydrogénées comme l'huile de jojoba hydrogénée ou l'huile de ricin hydrogénée ou résultant de la polymérisation de l'éthylène et/ou du propylène, les cires obtenues par synthèse de Fischer-Tropsch, les esters et les amides d'acides gras, les cires de silicone comme les alky-, alcoxy- et/ou esters de poly(di)méthylsiloxane solides à température ambiante, ayant de 10 à 45 atomes de carbone, des acides gras comme l'acide stéarique ou béhénique, et leurs mélanges.

7. Composition selon l'une quelconque des revendications précédentes, comprenant en outre au moins un composé choisi parmi les huiles, les cires non micronisées, les corps gras pâteux et leurs mélanges.

8. Composition selon la revendication précédente, dans laquelle les huiles sont choisies parmi les huiles volatiles ou non volatiles, hydrocarbonées d'origine animale, végétale ou minérale, les huiles synthétiques, les huiles siliconées, les huiles fluorées et leurs mélanges.

9. Composition selon l'une quelconque des revendications précédentes, se présentant sous une forme fluide.

10. Composition selon l'une quelconque des revendications précédentes, se présentant sous la forme d'un émulsion simple huile-dans-eau, ou eau-dans-huile, ou d'une émulsion multiple.

11. Composition selon la revendication précédente, se présentant sous la forme d'une émulsion eau-dans-huile.

12. Composition selon l'une quelconque des revendications précédentes, comprenant en outre au moins une matière colorante choisie parmi les matières colorantes pulvérulentes, telles que les pigments et les nacres, les colorants tels que les colorants lipophiles et les colorants hydrophiles, et leurs mélanges.

13. Composition selon la revendication précédente, dans laquelle les matières colorantes pulvérulentes sont traitées en surface avec un agent hydrophobe.

14. Composition selon la revendication précédente dans laquelle l'agent hydrophobe est choisi parmi le stéaroyl glutamate d'aluminium et le tri-isostréaroyl titanate d'isopropyle.

15. Composition selon l'une quelconque des revendications 12 à 14, dans laquelle les matières colorantes pulvérulentes sont présentes en une teneur allant de 0,5 à 30 % en poids, notamment allant de 5 à 20 % en poids, en particulier allant de 8 à 15 % en poids par rapport au poids total de la composition.

16. Composition selon l'une quelconque des revendications 12 à 15, dans laquelle les colorants lipophiles et/ou hydrophiles sont présents en une teneur allant de 0,0001 à 10 % en poids, de préférence de 0,01 à 5 % en poids, et encore plus préférentiellement de 0,1 à 3 % en poids, par rapport au poids total de la composition.

17. Composition selon l'une quelconque des revendications précédentes, destinée à être appliquée sur la peau et/ou les lèvres.

18. Composition selon l'une quelconque des revendications précédentes, se présentant sous forme de pâte ou de crème fluide comme les fonds de teint, les bases de maquillage de la peau ou les rouges à lèvres fluides, les eye-liners, les compositions de protection solaire ou de coloration de la peau ou encore de maquillage du corps.

19. Composition selon la revendication précédente se présentant sous la forme d'un fond de teint fluide ou d'une base de maquillage de la peau fluide.
